# EUROPÄISCHE PATENTANMELDUNG

(11) **EP 4 681 688 A1**
(43) Veröffentlichungstag der Anmeldung: **21.01.2026**
(21) Anmeldenummer: 24189725.5
(22) Anmeldetag: 19.07.2024
(51) Int. Cl.: A61F 13/15, A61F 13/511, A61F 13/514, A61F 13/53, A61F 13/51

(54) **MEHRLAGIGE ANORDNUNG FÜR EIN HYGIENEPRODUKT UND HYGIENEPRODUKT**

(71) Anmelder: Kelheim Fibres GmbH, 93309 Kelheim (DE)
(72) Erfinder: Wunder, Natalie, 93309 Kelheim (DE); Mayer, Dominik, 93339 Riedenburg (DE); Crnoja-Cosic, Marina, 93309 Kelheim (DE); Bernt, Ingo, 93053 Regensburg (DE)
(74) Vertreter: Schwarz & Partner Patentanwälte GmbH

(57) **Zusammenfassung**

Die Erfindung betrifft eine mehrlagige absorbierende Anordnung umfassend zumindest eine erste, eine zweite und eine dritte Lage, die jeweils aus textilen Materialien gebildet sind, die jeweils aus einem zu zumindest 80 Gew.% aus Cellulosefasern bestehenden Fasermaterialien gebildet sind. Das textile Material der zweiten Lage ist zumindest teilweise aus einem Mischgarn aus einer cellulosischen Faser mit rundem Querschnitt und einer cellulosischen Faser mit mehrschenkeligem Querschnitt und einem Anteil von 40 Gew.% und mehr am Mischgarn aufgebaut, wobei das Mischgarn eine Feinheit von NM 70 oder weniger. Das textile Material der dritten Lage ist zumindest teilweise aus einem Mischgarn aus einer cellulosischen Hohlfaser und einer cellulosischen Faser mit rundem Querschnitt aufgebaut, wobei der Anteil der einen cellulosischen Hohlfaser im Mischgarn der dritten Lage 30 Gew.% oder mehr beträgt und wobei das Mischgarn der dritten Lage eine Feinheit von NM 85 oder weniger aufweist.

## Beschreibung

Die vorliegende Erfindung betrifft eine mehrlagige absorbierende Anordnung für ein Hygieneprodukt und ein Hygieneprodukt umfassend die mehrlagige absorbierende Anordnung.

### Hintergrund der Erfindung

Auf dem Markt vorhandene Hygieneprodukte umfassen meist mehrlagige Anordnungen aus den Materialien Baumwolle, Polyester, Nylon, Polyamide, Merinowolle und/oder Polyurethan. Die mehrlagigen Anordnungen sind üblicherweise zumindest dreilagig. Die dem Körper zugewandte Lage, auch Topsheet genannt, nimmt Körperflüssigkeiten wie Blut, Urin und/oder Sekrete auf und leitet diese an die als Saugkern bezeichnete zweite Lage, welche die Flüssigkeit speichert, weiter. Die dritte Lage, auch Backsheet genannt, ist wasserundurchlässig und soll einen Durchtritt der aufgesaugten Flüssigkeit verhindern.

Beispielsweise beschreibt WO 2014/022832 A1 eine Schutzeinlage, die an einem Kleidungsstück angebracht werden kann, welche eine erste, innere Funktionsschicht aufweist, die eine feuchtigkeitstransportierende Faser umfasst. Die Schutzeinlage umfasst zudem eine zweite Schicht aus einem feuchtigkeitsabsorbierenden Material und eine dritte, äußere Funktionsschicht aus einem wenig atmungsaktiven, feuchtigkeitsbeständigen Material. Als feuchtigkeitsbeständiges Material wird ein Polyester-Polyurethan-Laminatgewebe vorgeschlagen.

US 2014/0025027 A1 bezieht sich auf eine waschbare Unterwäsche, die mindestens eine feuchtigkeitsabsorbierende Gewebeschicht mit einer körperberührenden Oberfläche und einer Absorptionskapazität von mindestens etwa 300 g/m² und mindestens eine feuchtigkeitsabweisende Schicht umfasst, die angrenzend an die mindestens eine feuchtigkeitsabsorbierende Schicht angeordnet ist und eine Außenfläche umfasst, die der körperberührenden Oberfläche gegenüberliegt. Als geeignete Materialien für die feuchtigkeitsabsorbierende Schicht werden unter anderem gewebte oder nicht gewebte Mikrofaser- oder Polymergestricke, Stoffe aus hydrophilen Fasern, absorbierende oder superabsorbierende Schäume, Fasern oder Pulver genannt.

CA 3 182 849 A1 offenbart eine siebenschichtige Inkontinenzunterwäsche mit einem Schrittbereich, in dem sich eine Naht durch die erste Lage, die sechste Lage und die siebte Lage, nicht aber durch die zweite bis fünfte Lage erstreckt. Die dritte Schicht kann dabei eine feuchtigkeitsleitende Schicht sein, die vierte Schicht ein absorbierendes Material umfassen und die fünfte Schicht wasserundurchlässig sein. Als mögliche absorbierende Materialien werden Gewebe, Naturgarn, synthetisches Garn, Wolle, Polypropylen, Nylon und Polyester angeführt.

Die Konstruktion der auf dem Markt vorhandenen Hygieneprodukte weist einige Nachteile auf: Durch die Verwendung von verschiedensten Materialien ist es kaum möglich, das jeweilige Produkt am Ende deren Lebenszyklus zu recyceln oder biologisch abzubauen. Auch führt die Verwendung verschiedenster synthetischer Fasern zur Freisetzung von Microplastik während des Waschens.

In den letzten Jahren ist der Marktanteil von wiederverwendbaren Hygieneprodukten wie beispielsweise Menstruationstassen oder Periodenunterwäsche stark angestiegen. Gründe für das Wachstum sind unter anderem das steigende Umweltbewusstsein von Konsumentinnen und gesetzliche Richtlinien wie beispielsweise die Single Use Plastic Directive in der Europäischen Union. Gemäß der Richtlinie müssen Plastik enthaltende Single-Use Hygieneprodukte speziell gekennzeichnet werden.

Im Artikel "Development of Sustainable Menstruation Pants using Speciality Viscose Fibres" (Lenzinger Berichte 97 (2022), 32-37) werden wiederverwendbare Periodenslips offenbart, welche eine mehrlagige Anordnung umfassen, deren Topsheet aus einer Biesenstrickstruktur aus Kombination aus 25 % Celliant^{®} Viskose und 75 % Danufil^{®} besteht. Celliant^{®} Viskose wird durch Einarbeitung eines Pulvers umfassend lumineszierende Nanopartikel-Photonenmarker-Mikrokapseln in Viskosefasern hergestellt. Bei Danufil^{®} handelt es sich um eine Standardviskosefaser. Als Saugkern wird eine Florschlingen-Strickstruktur aus 50 % Bramante und 50 % Viloft^{®} eingesetzt. Bramante ist eine Viskosehohlfaser und Viloft^{®} eine Viskoseflachfaser. Zwischen dem Topsheet und dem Saugkern ist eine Verteilerschicht, welche auch als Aquisition-Distribution-Layer (ADL) bezeichnet wird, aus 50 % Galaxy^{®} und 50% Danufil^{®} angeordnet. Galaxy^{®} ist eine trilobale Viskosefaser, deren Darstellung in der Druckschrift EP 0 301 874 A1 beschrieben wird. Als mögliche Materialien für das Backsheet werden Polymilchsäure, unter Spannung gewebte Gewebe aus Merinowolle oder nachhaltige Beschichtungen angeführt. Ähnliche Sachverhalte werden in "International Fiber Journal" 2022 (5), 44-47 und "Melliand Textilberichte" 2022 (1), 17-19 beschrieben.

Ein Nachteil vieler wiederverwendbarer Hygieneprodukte ist, dass diese nur bei bis zu 40 °C gewaschen werden können, da die verwendeten Materialien bei einer höheren Temperatur beschädigt werden. Niedrige Waschtemperaturen sind jedoch nicht ausreichend, um Bakterien zuverlässig abzutöten. Um diesen Nachteil zu beheben, werden häufig Biozide wie Silberchlorid eingesetzt. Diese können jedoch beim Waschen des Produkts ins Abwasser gelangen und in der Folge schädlich auf die Umwelt einwirken.

Ein weiterer wesentlicher Nachteil etablierter biologisch abbaubarer Hygieneprodukte ist deren deutlich geringere Leistungsfähigkeit im Vergleich zu nicht abbaubarer Single-Use Produkten, insbesondere im Hinblick auf die Ansaugzeit von Flüssigkeiten und das Rücknässen von absorbierter Flüssigkeit.

### Zusammenfassung der Erfindung

Aufgabe der vorliegenden Erfindung ist es daher, eine mehrlagige absorbierende Anordnung für ein Hygieneprodukt sowie ein Hygieneprodukt bereitzustellen, bei welchen die vorgenannten Nachteile vermieden sind. Insbesondere soll die Anordnung biologisch abbaubar sein und zugleich eine bessere Leistungsfähigkeit in Bezug auf die Ansaugzeit und das Rücknässen aufweisen.

Die Aufgabe wird durch eine mehrlagige absorbierende Anordnung gemäß dem diesbezüglichen unabhängigen Anspruch gelöst.

Die mehrlagige absorbierende Anordnung umfasst zumindest die folgenden drei Lagen aus textilem Material, welches textile Material jeweils aus Fasermaterialien gebildet ist:
a) eine bei Verwendung einem Körper zugewandte erste Lage;
b) eine an einer dem Körper abgewandten Seite der ersten Lage vorgesehene zweite Lage;
c) eine an einer dem Körper abgewandten Seite der zweiten Lage vorgesehene dritte Lage.

Das Fasermaterial der ersten, zweiten und dritten Lage besteht zu jeweils zumindest 80 Gew.% aus Cellulosefasern, wobei das textile Material der zweiten Lage zumindest teilweise aus einem Mischgarn aus zumindest einer cellulosischen Faser mit rundem Querschnitt und zumindest einer cellulosischen Faser mit mehrschenkeligem Querschnitt aufgebaut ist und wobei das Mischgarn der zweiten Lage eine Feinheit von NM 70 oder weniger aufweist. Erfindungsgemäß beträgt der Anteil der zumindest einen cellulosischen Faser mit mehrschenkeligem Querschnitt im Mischgarn der zweiten Lage 40 Gew.% oder mehr.

Das textile Material der dritten Lage ist zumindest teilweise aus einem Mischgarn aus zumindest einer cellulosischen Hohlfaser und zumindest einer cellulosischen Faser mit rundem Querschnitt aufgebaut, wobei der Anteil der zumindest einen cellulosischen Hohlfaser im Mischgarn der dritten Lage 30 Gew.% oder mehr beträgt und wobei das Mischgarn der dritten Lage eine Feinheit von NM 85 oder weniger aufweist.

Ein weiterer Aspekt der vorliegenden Erfindung ist die Bereitstellung eines Hygieneprodukts umfassend oder bestehend aus einer hierin beschriebenen mehrlagigen, absorbierenden Anordnung.

### Kurzbeschreibung der Figuren

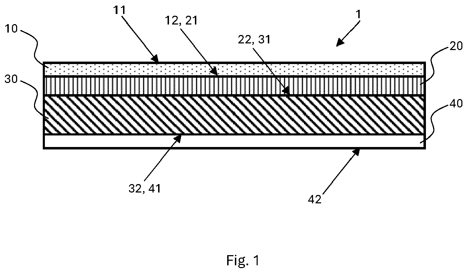

Figur 1 zeigt schematisch einen Querschnitt einer erfindungsgemäßen Anordnung 1 für ein Hygieneprodukt umfassend vier Lagen.

### Beschreibung der Ausführungsformen

Durch die Nutzung von cellulosischen Fasern in den Lagen der Anordnung ist die Anordnung zumindest größtenteils recyclebar und/oder biologisch abbaubar. Darüber hinaus zeichnet sich die Kombination der zumindest ersten, zweiten und dritten textilen Lage der Anordnung durch eine niedrige Ansaugzeit und niedriges Rücknässen aus.

Die Anordnung kann für ein Hygieneprodukt verwendet werden und ist insbesondere dazu geeignet Körperflüssigkeiten wie Urin, Blut und/oder Sekrete zu absorbieren.

In einer bevorzugten Ausführungsform bestehen die erste, zweite und/oder dritte textile Lage, bevorzugt jede Lage, zu zumindest 90 Gew.%, bevorzugt im Wesentlichen vollständig, besonders bevorzugt vollständig, aus Cellulosefasern. Im Wesentlichen vollständig bedeutet, dass die jeweilige Lage nicht-cellulosische Bestandteile wie weitere natürliche oder synthetische Fasern, Farbmittel, Duftstoffe und/oder Textilhilfsmittel aufweisen kann, welche die vorteilhaften Eigenschaften der Anordnung in Bezug auf biologische Abbaubarkeit und/oder Leistungsfähigkeit allenfalls geringfügig verschlechtern.

Vorzugsweise haben die nicht-cellulosischen Bestandteile in den jeweiligen Lagen einen Gesamtanteil von 10 Gew.% und weniger, bevorzugt 5 Gew.% und weniger.

Vorzugsweise ist das Mischgarn der zweiten Lage aus einer cellulosischen Faser mit rundem Querschnitt und einer cellulosischen Faser mit mehrschenkeligem Querschnitt aufgebaut.

Die zweite Lage wird in einer bevorzugten Ausführungsform vollständig aus dem Mischgarn aus der zumindest einen cellulosischen Faser mit rundem Querschnitt und der zumindest einen cellulosischen Faser mit mehrschenkeligem Querschnitt aufgebaut.

Ein Garn im Sinne der vorliegenden Erfindung ist jedes linienförmiges textiles Gebilde, das durch ein Spinnen der jeweiligen Fasern in üblichen Spinnverfahren gewonnen werden kann.

Der Begriff "runder Querschnitt" im Sinne der vorliegenden Erfindung umfasst auch Fasern, deren Querschnitt nicht ideal rund ist. Insbesondere fallen auch Standardviskosefasern mit deren typischen geriffelten (engl. "serrated") oder wolkenförmigen Querschnitt unter diesen Begriff. Der Querschnitt von Fasern kann mit üblichen optischen Messgeräten wie beispielsweise einem Lanameter bestimmt werden.

Vorzugsweise weist die zumindest eine cellulosische Faser mit mehrschenkeligem Querschnitt einen Y- (trilobalen), H-, T- oder X-förmigen Querschnitt auf.

Die zumindest eine mehrschenkelige cellulosische Faser im Mischgarn verleiht der zweiten Lage hervorragende Flüssigkeitstransport-Eigenschaften, sodass die zweite Lage vorzugsweise eine Verteilerlage (ADL) darstellt. Die Anwesenheit der zumindest einen Faser mit rundem Querschnitt im Mischgarn verringert ein Rücknässen, sodass der Tragekomfort gesteigert wird.

In einer bevorzugten Ausführungsform weist das Mischgarn der zweiten Lage eine Feinheit von NM 50 bis NM 10 auf. Die Einheit Nm gibt an, wie viele Meter des Mischgarns eine Masse von einem Gramm aufweist. Die Feinheit von Garnen kann gemäß DIN EN ISO 2060:1995-04 bestimmt werden.

Es ist bevorzugt, wenn der Anteil der zumindest einen cellulosischen Faser mit mehrschenkeligem, insbesondere trilobalem, Querschnitt im Mischgarn der zweiten Lage 45 bis 80 Gew.%, besonders bevorzugt 50 bis 65 Gew.%, beträgt. Dementsprechend wird der Rest des Mischgarns aus der cellulosischen Faser mit rundem Querschnitt und gegebenenfalls nicht-cellulosischen Bestandteilen wie oben definiert gebildet.

In einer weiteren Ausführungsform ist die dritte Lage vollständig aus dem Mischgarn aus der zumindest einen cellulosischen Hohlfaser und der zumindest einen cellulosischen Faser mit rundem Querschnitt aufgebaut.

Die dritte Lage eignet sich aufgrund der Kombination der zumindest einen cellulosischen Hohlfaser mit der zumindest einen cellulosischen Faser mit rundem Querschnitt für eine Verwendung als Saugkern, welcher die über die erste Lage aufgenommene und über die zweite Lage verteilte Körperflüssigkeit absorbiert und selbst unter Druck einen Flüssigkeitsaustritt verlässlich verhindert.

Zur Steigerung der maximalen Absorptionskapazität von Körperflüssigkeiten beträgt der Anteil der zumindest einen cellulosischen Hohlfaser im Mischgarn der dritten Lage bevorzugt 50 Gew.% oder mehr, insbesondere 60 Gew.% oder mehr. Dementsprechend wird der Rest des Mischgarns aus der cellulosischen Faser mit rundem Querschnitt und gegebenenfalls nicht-cellulosischen Bestandteilen wie oben definiert gebildet.

Vorzugsweise beträgt der maximale Anteil der zumindest einen cellulosischen Hohlfaser im Mischgarn der dritten Lage 85 Gew.%, besonders bevorzugt 75 Gew.%.

In einer bevorzugten Ausführungsform weist das Mischgarn der dritten Lage eine Feinheit von NM 50 bis NM 1, vorzugsweise bis NM 10, auf.

Das textile Material der ersten Lage kann zumindest teilweise, vorzugsweise vollständig, aus einem Garn aufgebaut sein, das im Wesentlichen aus zumindest einer, vorzugsweise einer, cellulosischen Faser mit rundem Querschnitt besteht und/oder eine Feinheit von NM 60 oder mehr, bevorzugt von NM 80 bis NM 180, besonders bevorzugt von NM 80 bis NM 150, ganz besonders bevorzugt von NM 85 bis NM 120, aufweist. Ein solches textiles Material zeichnet sich durch einen hohen Tragekomfort aus, sodass die erste Lage in der Anordnung die Funktion eines Topsheets übernehmen kann.

Der Begriff "im Wesentlichen" bedeutet im Hinblick auf das Garn der ersten Lage, dass das Garn nicht-cellulosische Bestandteile, insbesondere synthetische Fasern, aufweisen kann. Vorzugsweise beträgt der Anteil der nicht-cellulosischen Bestandteile zwischen 1 und 10 Gew.%, vorzugsweise zwischen 5 und 10 Gew.%. In einer bevorzugten Ausführungsform umfasst das Garn bis zu 10 Gew.% Elasthan, bevorzugt zwischen 2 und 7 Gew.-% Elasthan, besonders bevorzugt 5 Gew.% Elasthan.

Es ist bevorzugt, wenn die zumindest eine im textilen Material der ersten Lage eingesetzte Cellulosefaser mit rundem Querschnitt eine Feinheit von 0,7 bis 3,3 dtex, bevorzugt 1,0 bis 2,0 dtex, besonders bevorzugt 1,2 bis 1,4 dtex, aufweist.

Die Feinheit in der Einheit dtex gibt das Verhältnis von Masse je Länge an und kann gemäß DIN 60905-1, Teil 1, 1985-12 bestimmt werden. Ein dtex entspricht dabei einer Masse von 1 g pro 10.000 m.

Die hohe Feinheit der zumindest einen Cellulosefaser mit rundem Querschnitt der ersten Lage erlaubt die Bildung entsprechend feiner Garne, welche die vorteilhafte Aufnahmefähigkeit für Flüssigkeiten beibehalten.

In einer bevorzugten Ausführungsform weist die zumindest eine im textilen Material der zweiten Lage eingesetzte cellulosische Faser mit rundem Querschnitt eine Feinheit von 0,7 bis 4,2 dtex, bevorzugt 1,2 bis 1,4 dtex, besonders bevorzugt 1,3 dtex, und/oder die zumindest eine cellulosische Faser mit mehrschenkeligem, insbesondere trilobalem, Querschnitt eine Feinheit von 2,4 bis 4,2 dtex, bevorzugt 3,0 bis 3,6 dtex, besonders bevorzugt 3,3 dtex, auf. Bei diesen Feinheiten lassen sich die cellulosische Faser mit rundem Querschnitt und die cellulosische Faser mit mehrschenkeligem Querschnitt einfach zu dem Mischgarn für das textile Material der zweiten Lage verarbeiten, welches sich darüber hinaus durch vorteilhafte Eigenschaften bezüglich der Verteilung der aufgenommenen Flüssigkeit und des Verhinderns eines Rücknässens auszeichnet.

Die zumindest eine im textilen Material der dritten Lage eingesetzte cellulosische Hohlfaser kann eine Feinheit von 0,9 bis 5,5 dtex, bevorzugt 1,5 bis 3,5 dtex, besonders bevorzugt 2,1 dtex, aufweisen. Ergänzend oder alternativ kann die zumindest eine cellulosische Faser mit rundem Querschnitt in der dritten Lage eine Feinheit von 0,7 bis 6,7 dtex, bevorzugt 0,8 bis 1,5 dtex, besonders bevorzugt 0,9 dtex, aufweisen. Ein aus solchen Fasern gebildetes Mischgarn kann zu textilen Materialien verarbeitet werden, welche eine hohe Flüssigkeitsabsorptionskapazität aufweisen und zugleich biologisch abbaubar sind.

Das textile Material der ersten Lage kann ferner in Form eines Gestricks, insbesondere eines Single Jersey Gestricks, vorliegen. Ein solches textiles Material weist eine hohe Dehnbarkeit und Weichheit auf, woraus ein hoher Tragekomfort resultiert. Darüber hinaus nimmt das textile Material Körperflüssigkeiten schnell auf und eignet sich damit besonders für eine Verwendung als Topsheet in der Anordnung.

Optional können auch Henkel Gestrick-Bindungen in das Single Jersey Gestrick eingebracht werden. Des Weiteren kann das textile Material der ersten Lage zusätzlich perforiert werden, um die Flüssigkeitsdurchlässigkeit weiter zu erhöhen.

Vorzugsweise weist das Gestrick der ersten Lage, insbesondere das Single Jersey Gestrick, ein Flächengewicht von 60 g/m² bis 150 g/m², bevorzugt 70 g/m² bis 120 g/m², auf. Ein solch geringes Flächengewicht erlaubt die Herstellung mehrlagiger, absorbierender Anordnungen unter geringem Materialeinsatz und mit hohem Tragekomfort, ohne die vorteilhafte Funktionalität des Gestricks der ersten Lage in Bezug auf die Flüssigkeitsaufnahme zu beinträchtigen.

In einer bevorzugten Ausführungsform liegt das textile Material der zweiten Lage vorzugsweise in Form eines Gestricks, insbesondere eines Pique-Gestricks, vor. Die Ausgestaltung des textilen Materials der zweiten Lage als Gestrick führt zu einer effizienten Weiterleitung der von der ersten Lage aufgenommenen Flüssigkeit zur dritten Lage, sowie einer gleichmäßigen Verteilung der Oberfläche der dritten Lage. Darüber hinaus wird selbst bei einem Zusammendrücken der Anordnung ein unerwünschtes Rücknässen von der Anordnung auf den Körper effizient verhindert oder zumindest verringert, sodass der Tragekomfort deutlich gesteigert wird.

Vorzugsweise weist das Gestrick der zweiten Lage ein Flächengewicht von 70 bis 300 g/m², bevorzugt 100 g/m² bis 200 g/m², besonders bevorzugt 130 bis 160 g/m², auf.

Das textile Material der dritten Lage kann in Form eines Gestricks, insbesondere eines Single Jersey Gestricks oder eines Frottiergestricks, vorliegen. Die Ausgestaltung des textilen Materials der dritten Lage als Gestrick, insbesondere als Frottiergestrick, führt zu einer Steigerung des Flüssigkeitsrückhaltevermögens.

Vorzugsweise weist das Gestrick der dritten Lage ein Flächengewicht von 100 g/m² bis 500 g/m², insbesondere 200 g/m² bis 350 g/m², auf.

In einer bevorzugten Ausführungsform sind die in der ersten, zweiten und/oder dritten Lage eingesetzten Cellulosefasern nach dem Viskoseverfahren hergestellte Regeneratfasern. Besonders bevorzugt sind alle in den Lagen eingesetzten Cellulosefasern nach dem Viskoseverfahren hergestellte Regeneratfasern.

Üblicherweise umfasst das Viskoseverfahren die Stufen
i) Erzeugung der Spinnlösung;
ii) Erspinnen der Filamente; und
iii) Nachbehandlung der ersponnenen Fäden.

Ein wesentlicher Vorteil des Viskoseverfahrens ist, dass der als Ausgangsmaterial verwendete Zellstoff aus unterschiedlichen nachwachsenden Rohstoffen, insbesondere aus Hölzern wie Buchen, Fichten und/oder Bambus, gewonnen werden kann und als Produkt Viskosefasern mit wohldefinierten Eigenschaften erhalten werden.

Die für das textile Material der ersten, zweiten und/oder dritten Lage verwendeten Cellulosefasern können durch entsprechende Modifikationen der Verfahrensführung und Kontrolle der Verfahrensbedingungen gewonnen werden.

Querschnittsmodifizierte Fasern, wie mehrschenkelige Fasern, können durch Verwendung entsprechend profilierten Spinndüsen erhalten werden. Beispielsweise ist die Herstellung trilobaler Cellulosefasern aus EP 0 301 874 A1 bekannt.

Die Herstellung von Hohlfasern geschieht durch Zugabe von Natriumcarbonat zur Spinnlösung und ist beispielsweise aus US 4,129,679 A bekannt.

Alternativ oder ergänzend können die in der ersten, zweiten und/oder dritten Lage eingesetzten Cellulosefasern nach dem Lyocellverfahren hergestellte Fasern sein.

Die absorbierende, mehrlagige Anordnung kann
d) eine an einer dem Körper abgewandten Seite der dritten Lage vorgesehene vierte Lage aufweisen.

Die vierte Lage besteht vorzugsweise aus einem wasserundurchlässigen oder wasserabweisenden Material oder umfasst dieses. Im Sinne der Erfindung sind Materialien wasserabweisend, wenn der Wassereintrittsdruck bestimmt nach DIN EN 20811:1992 zumindest 4000 mm Wassersäule beträgt, und wasserundurchlässig, wenn der Wassereintrittsdruck zumindest 8000 mm Wassersäule beträgt.

Das wasserundurchlässige oder wasserabweisende Material ist insbesondere eine Polymermembran oder eine Beschichtung. Unter Membran wird eine flächige Struktur verstanden, welche im Verhältnis zu ihrer Dicke eine große flächige Ausdehnung hat.

In einer bevorzugten Ausführungsform ist das wasserundurchlässige oder wasserabweisende Material biologisch abbaubar. Vorzugsweise werden biologisch abbaubare Kunststoffe, insbesondere Polymilchsäure (PLA) eingesetzt. Vorzugsweise wird eine PLA-Folie als vierte Lage verwendet.

Der Querschnitt einer möglichen Ausführungsform einer solchen vierlagigen, absorbierenden Anordnung 1 ist in Fig. 1 gezeigt.

Die Anordnung 1 umfasst eine erste Lage 10, welche bei Verwendung der Anordnung eine dem Körper einer Person (nicht gezeigt) zugewandte erste Oberfläche 11 aufweist. Vorzugsweise steht die Anordnung 1 über die erste Oberfläche 11 der ersten Lage 10 im unmittelbaren Kontakt mit dem Körper, insbesondere in unmittelbaren Kontakt mit dem Intimbereich der Person. Die erste Lage 10 weist eine der ersten Oberfläche 11 abgewandte zweite Oberfläche 12 auf.

Die erste Lage 10 kann aus jedem hierin beschriebenen textilen Material der ersten Lage bestehen oder dieses umfassen. Abgesonderte Körperflüssigkeiten werden von der ersten Lage 10 über die erste Oberfläche 11 aufgenommen und vom Körper weggeleitet.

Die erste Lage 10 kann ein Flächengewicht von 60 g/m² bis 150 g/m², bevorzugt 70 g/m² bis 120 g/m², aufweisen. Die erste Lage kann perforiert sein (nicht gezeigt).

Die Anordnung umfasst des Weiteren eine zweite Lage 20, welche eine erste Oberfläche 21 und eine zweite Oberfläche 22 aufweist. Die zweite Lage 20 steht über die erste Oberfläche 21 mit der zweiten Oberfläche 12 der ersten Lage 10, vorzugsweise unmittelbar, in Kontakt.

Die zweite Lage 20 kann dabei aus jedem hierin beschriebenen textilen Material der zweiten Lage bestehen.

Die zweite Lage 20 kann ein Flächengewicht von 70 bis 300 g/m², bevorzugt 100 g/m² bis 200 g/m², besonders bevorzugt 130 bis 160 g/m², aufweisen.

Von der ersten Lage 10 aufgenommene Körperflüssigkeit geht auf die zweite Lage 20 über und wird einer dritten Lage 30 zugeführt. Die zweite Lage 20 übernimmt dabei die Funktion einer ADL in der Anordnung 1.

Die dritte Lage 30 steht über eine erste Oberfläche 31 der dritten Lage 30 mit der zweiten Oberfläche 22 der zweiten Lage 20, vorzugsweise unmittelbar, in Kontakt.

Die dritte Lage 30 kann dabei aus jedem hierin beschriebenen textilen Material der dritten Lage bestehen.

Die dritte Lage 30 kann ein Flächengewicht von 100 g/m² bis 500 g/m², insbesondere 200 g/m² bis 350 g/m², aufweisen. Die dritte Lage 30 übernimmt insbesondere die Funktion eines Saugkerns in der Anordnung 1 und bestimmt maßgeblich deren maximale Flüssigkeitsabsorptionskapazität.

Die Anordnung kann des Weiteren eine an einer der ersten Oberfläche 31 abgewandten zweiten Oberfläche 32 der dritten Lage 30 angeordnete vierte Lage 40 aufweisen. Die vierte Lage umfasst eine der dritten Schicht 30 zugewandte erste Oberfläche 41, über welche sie vorzugsweise mit der dritten Schicht in unmittelbaren Kontakt steht.

Die vierte Lage 40 ist vorzugsweise eine Schicht oder Beschichtung bestehend aus einem hierin beschriebenen wasserabweisenden oder wasserundurchlässigen Material oder umfasst ein solches. Insbesondere kann die vierte Lage 40 eine PLA-Folie sein. Auf diese Weise kann ein Durchtritt der in der dritten Lage absorbierten Körperflüssigkeit nach außen unterbunden werden.

Vorzugsweise stellt die vierte Lage 40 die äußerste (körperfernste) Lage der Anordnung 1 dar.

Die einzelnen Lagen 10, 20, 30, und/oder 40 der Anordnung 1 können beispielsweise miteinander vernäht, verklebt und/oder verschweißt sein, um einen Zusammenhalt der Anordnung 1 zu gewährleisten. In einer bevorzugten Ausführungsform (nicht gezeigt) sind die beiden äußeren Lagen, beispielsweise Lage 10 und Lage 40, entsprechend größer als die inneren Lagen 20 und 30 dimensioniert, sodass diese miteinander vernäht und die Lagen 20 und 30 umschließen.

Die Anordnung kann weitere Lagen (nicht gezeigt) umfassen. Beispielsweise können dies weitere absorbierende Lagen sein, die zwischen der zweiten Schicht 20 und der dritten Schicht 30 und/oder der dritten Schicht 30 und der vierten Schicht 40 sein. Diese weiteren Lagen sind insbesondere ebenfalls biologisch abbaubar und für wiederverwendbare Anordnungen geeignet.

Die vorliegende Aufgabe wird des Weiteren durch ein Hygieneprodukt gemäß dem diesbezüglichen unabhängigen Anspruch gelöst.

Das Hygieneprodukt umfasst eine mehrlagige, absorbierende Anordnung oder besteht aus dieser. Die mehrlagige, absorbierende Anordnung kann jede hierin beschriebene mehrlagige, absorbierende Anordnung sein.

In einer bevorzugten Ausführungsform sind mögliche weitere Bestandteile des Hygieneprodukts ebenfalls biologisch abbaubar. Vorzugsweise besteht das Hygieneprodukt vollständig aus textilen Materialien auf Basis cellulosischer Fasern.

Das Hygieneprodukt ist vorzugsweise wiederverwendbar. Eine Reinigung des Hygieneprodukts ist insbesondere durch Waschen bei einer Temperatur von 60°C möglich. Dies hat den Vorteil, dass auf die Verwendung eines Biozids verzichtet werden kann.

Das Hygieneprodukt kann eine Binde, eine Einlage, eine Windel, insbesondere Babywindel, oder eine Periodenunterwäsche ist. Das Hygieneprodukt ist insbesondere für den Einsatz bei leichter Inkontinenz (50-100 ml in bis zu 4 Stunden, Tröpfchen bis kleine Urinmengen) eingerichtet.

### Beispiele

Die nachfolgenden Beispiele erläutern die Erfindung, ohne diese zu beschränken.

### Herstellung der erfindungsgemäßen Anordnung B1:

Die erste Lage besteht aus einer cellulosischen Rundfaser (Standardviskosefaser Danufil ^{®}) der Faserfeinheit 1,3 dtex und der Faserlänge 40 mm, welche mithilfe des Ringspinnverfahrens zu einem Garn der Garnfeinheit NM 100/1 versponnen und anschließend zu einem Single-Jersey Gestrick weiterverarbeitet wird. Das Gestrick wird auf einer Rundstrickmaschine hergestellt und hat ein Flächengewicht von 70 g/m².

Die zweite Lage besteht zu 50 Gew.% aus einer cellulosischen Faser mit trilobalem Querschnitt der Faserfeinheit 3,3 dtex (Galaxy ^{®}) und der Faserlänge 38 mm und zu 50 Gew.% aus einer cellulosischen Rundfaser (Standardviskosefaser Danufil ^{®}) der Faserfeinheit 1,3 dtex und der Faserlänge 40 mm. Aus den genannten Fasern wird mithilfe des Ringspinnverfahrens ein Garn der Garnfeinheit NM 36/1 gesponnen und anschließend auf einer Rundstrickmaschine ein Pique-Gestrick mit einem Flächengewicht von 150 g/m² hergestellt.

Die dritte Lage besteht zu 60 Gew.% aus einer cellulosischen Hohlfaser der Faserfeinheit 2,1 dtex und der Faserlänge 40 mm (Typ Bramante) und zu 40 % aus einer cellulosischen Rundfaser (Standardviskosefaser) mit der Faserfeinheit 0,9 dtex (Typ Viseta^{®}) und der Faserlänge 40 mm. Aus den genannten Fasern wird mithilfe des Ringspinnverfahrens ein Garn der Garnfeinheit NM 20/1 gesponnen und anschließend auf einer Rundstrickmaschine ein Frottier Gestrick mit einem Flächengewicht von 290 g/m² hergestellt.

Als vierte Lage wird eine PLA-Folie verwendet.

Die vier Lagen werden durch Vernähen miteinander verbunden.

### Herstellung der erfindungsgemäßen Anordnung B2:

Die erste Lage besteht aus einer cellulosischen Rundfaser (Standardviskosefaser) der Faserfeinheit 0,9 dtex (Typ Viseta^{®}) und der Faserlänge 40 mm mit 5 Gew.% Elasthan, welche mithilfe des Ringspinnverfahrens zu einem Garn der Garnfeinheit NM 120/1 versponnen und anschließend zu einem Single-Jersey Gestrick weiterverarbeitet werden. Das Gestrick wird auf einer Rundstrickmaschine hergestellt und hat ein Flächengewicht von 120 g/m2.

Die zweite Lage besteht zu 50 Gew.% aus einer cellulosischen Faser mit trilobalem Querschnitt (Galaxy^{®}) der Faserfeinheit 3,3 dtex und der Faserlänge 38 mm und zu 50 Gew.% aus einer cellulosischen Rundfaser der Faserfeinheit 1,3 dtex (Danufil ^{®}) und der Faserlänge 40 mm. Aus den genannten Fasern wird mithilfe des Ringspinnverfahrens ein Garn der Garnfeinheit NM 36/1 gesponnen und anschließend auf einer Rundstrickmaschine ein Pique Gestrick mit einem Flächengewicht von 150 g/m² hergestellt.

Die dritte Lage besteht zu 60 Gew.% einer cellulosischen Hohlfaser (Typ Bramante) der Faserfeinheit 2,1 dtex und der Faserlänge 40 mm und zu 40 Gew.% einer cellulosischen Rundfaser der Faserfeinheit 0,9 dtex (Typ Viseta ^{®}) und der Faserlänge 40 mm. Aus den genannten Fasern wird mithilfe des Ringspinnverfahrens ein Garn der Garnfeinheit NM 20/1 gesponnen und anschließend auf einer Rundstrickmaschine ein Frottier Gestrick mit einem Flächengewicht von 290 g/m² hergestellt.

Als vierte Lage wird eine PLA-Folie verwendet.

Die vier Lagen werden durch Vernähen miteinander verbunden.

Die Materialzusammensetzungen der einzelnen Lagen der Referenzbeispiele A1 bis A9 sowie der erfindungsgemäßen Beispielen B1 und B2 sind in der Tab. 1 zusammengefasst.

**Tab. 1: Materialzusammensetzungen der Lagen**

| **Bsp.** | **Lage 1** | **Lage 2** | **Lage 3** | **Lage 4** |
|---|---|---|---|---|
| A1 | 97% Bio-Baumwolle + 3% Elastan | - | 100% Tencel (Lyocell) | Polyester mit Polyurethanbeschichtung |
| A2 | 67% Polyester + 33% Modal | - | 80% Baumwolle + 20% Polyester | Polyurethanbesch ichtung auf der Rückseite von Lage 2 |
| A3 | 100 % Polyester | - | 100% Bambus-Viskose | Polyester mit Polyurethanbeschichtung |
| A4 | 95% Baumwolle + 5% Elastan | - | 95% Baumwolle + 5% Elastan | 100 % Polyurethan |
| A5 | 100% Bio-Baumwolle | - | 100% Polyester | 100% Polyester |
| A6 | 100% Bio-Baumwolle | - | 80% Bambus-Viskose + 20% Polyester | 67% Polyester + 33% Polyurethan |
| A7* | 100% Merinowolle | - | 80% Baumwolle + 20% Polyester | Polyester mit Polyurethanbeschichtung |
| A8 | 95% Bio-Baumwolle + 5% Elastan | - | 80% Polyester + 20% Elastan | 100% thermoplastisches Polyurethan (TPU) |
| B1 | 100% Rundfaser (Single-Jersey) | 50% Galaxy^{®} + 50% Danufil^{®} (Pique) | 60% Bramante | PLA-Folie |
| | | | 40% Viseta (Single Jersey) | |
| B2 | 95% Rundfaser + 5% Elasthan (Single-Jersey) | 50% Galaxy^{®} + 50% Danufil^{®} (Pique) | 60% Bramante | PLA-Folie |
| | | | 40% Viseta (Frottier) | |

| | | | | |
|---|---|---|---|---|
| * ...umfasst drei Lagen 3 mit derselben Zusammensetzung | | | | |

Die Referenzbeispiele A1 bis A8 entsprechen mehrlagigen, absorbierenden Anordnungen, welche bereits auf dem Markt vertrieben werden. Lage 1 der jeweiligen Anordnung entspricht dabei dem Topsheet, Lage 2 der Verteilerschicht, Lage 3 dem Saugkern und Lage 4 dem Backsheet.

### Methoden:

Die Leistungsfähigkeit der einzelnen Beispielsanordnungen wurde durch Bestimmung des Wasserrückhaltevermögens, der Ansaugzeit, des Rewet-Werts sowie der maximalen Absorptionskapazität gemäß den nachfolgend angeführten Methoden bestimmt. Alle Tests wurden in einem klimatisierten Raum mit einer Temperatur von etwa 23 +/- 2 °C und einer relativen Luftfeuchtigkeit von etwa 50 +/- 2 % durchgeführt.

### Wasserrückhaltevermögen:

Die Bestimmung des Wasserrückhaltevermögens der Anordnungen basiert auf der Methode EDANA NWSP 241.0.R2 (15) Polyacrylat-Superabsorber-Pulver-Bestimmung des Flüssigkeitsrückhaltevermögens einer Kochsalzlösung durch gravimetrische Messung nach Zentrifugation. Diese wird jedoch auf die saugfähigen Anordnungen gemäß der Prüfmethode PA07/05 der Hygiene-Technologie GmbH (Hy-Tec), Haan, Deutschland, angewendet.

Es wird zunächst das Trockengewicht des jeweiligen Prüfmusters durch Wägung bestimmt.

Das Prüfmuster wird dann für 30 min in einer Kochsalzlösung (0,9 Gew.% NaCl) gelagert. Das gequollene Prüfmuster wird für 3 Minuten in einer Zentrifuge (Firma Heraeus Sepatech GmbH, Innendurchmesser 225 mm, 1400 U/min) mit einer Zentrifugalkraft von 250 g zentrifugiert. Das Prüfmuster wird entnommen und gewogen. Aus der Gewichtsdifferenz zum Trockengewicht kann Wasserrückhaltevermögen in Bezug auf das Trockengewicht in % berechnet werden.

### Ansaugzeit und Rewet-Bestimmung:

Die Bestimmung der Ansaugzeit sowie des Rewet-Werts basiert auf der Nonwoven Adult Incontinence Produkte: Rate of Acquisition and Re-Wet Test (EDANA Testmethode NWSP 070.9.R1 (15)), die nach der Testmethode PA07/03 der Hygiene-Technologie GmbH (Hy-Tec), Haan, Deutschland modifiziert ist.

Bei der Prüfung wird eine durchbrochene Plexiglasplatte (70 mm x 220 mm) mit einem zentralen Zylinder von 20 mm Innendurchmesser und 130 mm Höhe mit einem Gewicht von 255 g verwendet. Die Platte ist so ausgestaltet, dass zwei Gewichte von je 1 kg auf die Platte auf beide Seiten des Zylinders gelegt werden können. Nach dem Aufsetzen des Zylinders, der Platte sowie den zentrierten Gewichten in der Mitte der Platte auf das jeweilige Prüfmuster werden 15 ml einer Kochsalzlösung (0,9 Gew.-% NaCl) als synthetischer Urin in den Zylinder gegeben, und die Zeit bis zur vollständigen Aufnahme der Flüssigkeit gemessen, welche der Ansaugzeit entspricht.

Nach 30 Sekunden werden die Gewichte, die Platte und der Zylinder entfernt. Sechs Lagen abgewogenes Filterpapier (je 110 mm x 110 mm) werden auf das Prüfmuster gelegt, direkt gefolgt von einem 1 kg Gewicht. Nach 10 Sekunden wird das Gewicht entfernt und das Gewicht der Filterpapiere bestimmt. Das Differenzgewicht in Gramm entspricht dem Rewet-Wert.

### Maximale Absorptionskapazität nach Hohenstein:

Eine Prüfflüssigkeit wird durch Lösen von 8 g CMC (Carboxy Methyl Cellulose, 0,60 - 0,95 Substitutionsgrad, CAS 9004-32-4, Sigma Aldrich) und 9 g NaCl in einem Liter destilliertem Wasser unter Rühren und Erhitzen auf 35 °C und anschließendem Abkühlen auf Raumtemperatur hergestellt.

Für die Durchführung der Prüfung wird ein vom Hohenstein Institut entwickelter Prüfaufbau verwendet, welcher aus einer Halfpipe, einem transparenten Stempel mit Öffnung und einem nicht transparenten Stempel ohne Öffnung besteht.

Zur Probenvorbereitung werden die Prüfmuster vor dem Testen bei 30°C mit Feinwaschmittel gewaschen, um Prozesschemikalien und dergleichen zu entfernen, und etwaige Seitennähte aufgetrennt, sodass das jeweilige Prüfmuster ideal in der Halfpipe platziert werden kann.

Zuerst wird eine Lage Filterpapier gewogen (+/- 0,01 g) und in der Halfpipe abgelegt. Anschließend wird das trockene Prüfmuster (z.B. Menstruationsunterwäsche) gewogen und auf dem Filterpapier in der Halfpipe platziert. Zur Detektion des Auslaufens muss das Filterpapier auf beiden Seiten des absorbierenden Bereichs des Prüfmusters herausragen. Der mit Gewichten beschwerte transparente Stempel (Gesamtmasse: 1kg) wird auf dem Muster platziert.

Ein Prüfflüssigkeitsvolumen von 3 ml wird über eine Dauer von 10 Sekunden mit einer Pipette durch die Öffnung des transparenten Stempels auf das Prüfmuster aufgetragen. Nach 5 Minuten wird der transparente Stempel mit den darauf platzierten Gewichten für 5 Sekunden komplett abgehoben und es findet eine Sichtkontrolle auf ausgetretene Flüssigkeit statt. Danach wird der transparente Stempel mit den Gewichten erneut auf dem Prüfmuster platziert. Dieser Vorgang wird so oft wiederholt, bis auf dem Filterpapier unter dem Prüfmuster ein Auslaufen der Prüfflüssigkeit beobachtet werden kann. Das Gesamtvolumen der aufgetragenen Prüfflüssigkeit entspricht der maximalen Absorptionskapazität in ml.

Die Messung wird 3 mal pro Muster wiederholt und ein Mittelwert berechnet.

### Resultate:

### Wasserrückhaltevermögen:

Das Wasserrückhaltevermögen erlaubt einen Vergleich der Leistungsfähigkeit der einzelnen Saugkörper (Lage 3) und ist besonders für eine Anwendung der mehrlagigen, absorbierenden Anordnungen in wiederverwendbaren Hygieneprodukten relevant. Durch die Zentrifugation wird Flüssigkeit, welche nur lose zwischen den Fasern aufgenommen wurde, wieder entfernt. Dies simuliert jene Belastungen, welchen die Anordnungen bei deren Anwendung aufgrund von Druck und Bewegung ausgesetzt sind.

Die ermittelten Werte für das Wasserrückhaltevermögen sowie die Flächengewichte der jeweiligen textilen Fläche sind in Tab. 2 zusammengefasst. Die erfindungsgemäßen Anordnungen B1 und B2 weisen jeweils ein Wasserrückhaltevermögen von 111,6 % auf, was deutlich über den Werten der Referenzbeispiele A1 bis A8 liegt.

**Tab.2: Wasserrückhaltevermögen und Flächengewichte der Lage 3**

| **Beispiel** | **Wasserrückhaltevermögen in %** | **Flächengewicht in g/m²** |
|---|---|---|
| A1 | 54,9 | 240 |
| A2 | 35,4 | 152 |
| A3 | 67,3 | 357 |
| A4 | 38,9 | 170 |
| A5 | 8,7 | 255 |
| A6 | 69,0 | 261 |
| A7 | 35,3 | 553 |
| A8 | 10,7 | 250 |
| B1 | 111,6 | 290 |
| B2 | 111,6 | 290 |

### Rewet-Werte und Ansaugzeit:

In Tab. 3 sind die ermittelten Rewet-Werte und Ansaugzeiten gegenübergestellt. Die erfindungsgemäßen Anordnungen weisen im Vergleich zu den Referenzbeispielen A1 bis A8 die niedrigsten Rewet-Werte bei gleichzeitig niedriger Ansaugzeit auf. Dies bedeutet, dass die erfindungsgemäßen Anordnungen Körperflüssigkeiten schnell aufnehmen können und aufgenommene Flüssigkeit verlässlich zurückhalten, sodass ein hoher Tragekomfort resultiert.

**Tab.3: Rewet-Werte und Ansaugzeiten**

| **Beispiel** | **Rewet in g** | **Ansaugzeit in s** |
|---|---|---|
| A1 | 6,080 | 7,324 |
| A2 | 6,308 | 6,982 |
| A3 | 4,886 | 7,110 |
| A4 | 6,200 | 11,524 |
| A5 | 6,140 | 6,772 |
| A6 | 4,470 | 5,116 |
| A7 | 3,574 | 3,682 |
| A8 | 4,948 | 4,682 |
| B1 | 3,010 | 5,300 |
| B2 | 3,040 | 4,460 |

### Maximale Absorptionskapazität:

In Tab. 4 sind die ermittelten maximalen Absorptionskapazitäten zusammengefasst. Die erfindungsgemäßen Anordnungen B1 und B2 weisen im Vergleich zu den Referenzbeispielen A1 bis A8 eine deutlich höhere Absorptionskapazität auf.

**Tab.4: Maximale Absorptionskapazitäten**

| **Beispiel** | **Maximale Flüssigkeitsaufnahme in ml** |
|---|---|
| A1 | 6 |
| A2 | 6 |
| A3 | 9 |
| A4 | 5 |
| A5 | 3 |
| A6 | 6 |
| A7 | 8 |
| A8 | 4,5 |
| B1 | 15 |
| B2 | 12 |

In Zusammenschau zeigen die experimentellen Resultate, dass die erfindungsgemäßen Anordnungen in Bezug auf deren Leistungsfähigkeit den aktuell am Markt erhältlichen Produkten überlegen sind. Unter Berücksichtigung, dass die erfindungsgemäßen Anordnungen zum Großteil aus cellulosischem Material bestehen, wurde zugleich die Nachhaltigkeit gesteigert.

### Bezugszeichenliste

- 1: Anordnung
- 10: erste Lage
- 11, 12: Oberfläche der ersten Lage
- 20: zweite Lage
- 21, 22: Oberfläche der zweiten Lage
- 30: dritte Lage
- 31, 32: Oberfläche der dritten Lage
- 40: vierte Lage
- 41, 42: Oberfläche der vierten Lage

## Patentansprüche

1. Mehrlagige absorbierende Anordnung (1) für ein Hygieneprodukt, die Anordnung (1) umfassend zumindest die folgenden drei Lagen (10, 20, 30) aus textilem Material, welches textile Material jeweils aus Fasermaterialien gebildet ist:
a) eine bei Verwendung der Anordnung (1) einem Körper zugewandte erste Lage (10);
b) eine an einer dem Körper abgewandten Seite der ersten Lage vorgesehene zweite Lage (20);
c) eine an einer dem Körper abgewandten Seite der zweiten Lage (20) vorgesehene dritte Lage (30);
wobei das Fasermaterial der ersten, zweiten und dritten Lage (10, 20, 30) zu jeweils zumindest 80 Gew.%, bevorzugt im Wesentlichen vollständig, aus Cellulosefasern besteht und wobei
das textile Material der zweiten Lage (20) zumindest teilweise, vorzugsweise vollständig, aus einem Mischgarn aus zumindest einer cellulosischen Faser mit rundem Querschnitt und zumindest einer cellulosischen Faser mit mehrschenkeligem, insbesondere trilobalem, Querschnitt aufgebaut ist,
wobei das Mischgarn der zweiten Lage (20) eine Feinheit von NM 70 oder weniger, bevorzugt NM 50 bis NM 10, aufweist,
wobei ein Anteil der zumindest einen cellulosischen Faser mit mehrschenkeligem, insbesondere trilobalem, Querschnitt im Mischgarn der zweiten Lage b) 40 Gew.% oder mehr beträgt,
wobei das textile Material der dritten Lage (30) zumindest teilweise, vorzugsweise vollständig, aus einem Mischgarn aus zumindest einer cellulosischen Hohlfaser und zumindest einer cellulosischen Faser mit rundem Querschnitt aufgebaut ist,
wobei der Anteil der zumindest einen cellulosischen Hohlfaser im Mischgarn der dritten Lage (30) 30 Gew.% oder mehr, bevorzugt 50 Gew.% oder mehr, insbesondere 60 Gew.% oder mehr, beträgt und
wobei das Mischgarn der dritten Lage (30) eine Feinheit von NM 85 oder weniger, bevorzugt NM 50 bis NM 1, aufweist.

2. Anordnung (1) gemäß Anspruch 1, **dadurch gekennzeichnet, dass** das textile Material der ersten Lage (10) zumindest teilweise, vorzugsweise vollständig, aus einem Garn aufgebaut ist, das im Wesentlichen aus zumindest einer cellulosischen Faser mit rundem Querschnitt besteht und/oder dass das Garn der ersten Lage (10) eine Feinheit von NM 60 oder mehr, bevorzugt NM 80 bis NM 180, besonders bevorzugt NM 85 bis NM 120, aufweist.

3. Anordnung (1) gemäß Anspruch 2, **dadurch gekennzeichnet, dass** die zumindest eine im textilen Material der ersten Lage (10) eingesetzte Cellulosefaser mit rundem Querschnitt eine Feinheit von 0,7 bis 3,3 dtex, bevorzugt 1,0 bis 2,0 dtex, besonders bevorzugt 1,2 bis 1,4 dtex, aufweist.

4. Anordnung (1) gemäß einem der vorhergehenden Ansprüche, **dadurch gekennzeichnet, dass** die zumindest eine im textilen Material der zweiten Lage (20) eingesetzte cellulosische Faser mit rundem Querschnitt eine Feinheit von 0,7 bis 4,2 dtex, bevorzugt 1,3 dtex, und/oder die zumindest eine cellulosische Faser mit mehrschenkeligem, insbesondere trilobalem, Querschnitt eine Feinheit von 2,4 bis 4,2 dtex, bevorzugt 3,3 dtex, aufweist.

5. Anordnung (1) gemäß einem der vorhergehenden Ansprüche, **dadurch gekennzeichnet, dass** die zumindest eine im textilen Material der dritten Lage (30) eingesetzte cellulosische Hohlfaser eine Feinheit von 0,9 bis 5,5 dtex, bevorzugt 2,1 dtex, und/oder die zumindest eine cellulosische Faser mit rundem Querschnitt eine Feinheit von 0,7 bis 6,7 dtex, bevorzugt 0,9 dtex, aufweist.

6. Anordnung (1) gemäß einem der vorhergehenden Ansprüche, **dadurch gekennzeichnet, dass** das textile Material der ersten Lage (10) in Form eines Gestricks, insbesondere eines Single Jersey Gestricks, vorliegt.

7. Anordnung (1) gemäß Anspruch 6, **dadurch gekennzeichnet, dass** das Gestrick der ersten Lage (10) ein Flächengewicht von 60 g/m² bis 150 g/m², bevorzugt 70 g/m² bis 120 g/m², aufweist.

8. Anordnung (1) gemäß einem der vorhergehenden Ansprüche, **dadurch gekennzeichnet, dass** das textile Material der zweiten Lage (20) in Form eines Gestricks, insbesondere eines Pique-Gestricks, vorliegt.

9. Anordnung (1) gemäß Anspruch 8, **dadurch gekennzeichnet, dass** das Gestrick der zweiten Lage (20) ein Flächengewicht von 70 bis 300 g/m², bevorzugt 100 g/m² bis 200 g/m², besonders bevorzugt 130 bis 160 g/m², aufweist.

10. Anordnung (1) gemäß einem der vorhergehenden Ansprüche, **dadurch gekennzeichnet, dass** das textile Material der dritten Lage (30) in Form eines Gestricks, insbesondere eines Single Jersey Gestricks oder eines Frottiergestricks, vorliegt.

11. Anordnung (1) gemäß Anspruch 10, **dadurch gekennzeichnet, dass** das Gestrick der dritten Lage (30) ein Flächengewicht von 100 g/m² bis 500 g/m², insbesondere 200 g/m² bis 350 g/m², aufweist.

12. Anordnung (1) gemäß einem der vorhergehenden Ansprüche, **dadurch gekennzeichnet, dass** die in der ersten, zweiten und/oder dritten Lage (10, 20, 30) eingesetzten Cellulosefasern nach dem Viskoseverfahren hergestellte Regeneratfasern sind.

13. Anordnung (1) gemäß einem der vorhergehenden Ansprüche, **dadurch gekennzeichnet, dass** die Anordnung (1)
d) eine an einer dem Körper abgewandten Seite der dritten Lage (30) vorgesehene vierte Lage (40) aufweist,
wobei die vierte Lage (40) vorzugsweise aus einem wasserundurchlässigen oder wasserabweisenden Material, insbesondere einer Polymermembran, besteht oder dieses umfasst.

14. Hygieneprodukt, **dadurch gekennzeichnet, dass** das Hygieneprodukt eine Anordnung (1) gemäß einer der Ansprüche 1 bis 13 umfasst oder aus dieser besteht, wobei das Hygieneprodukt bevorzugt wiederverwendbar ist und/oder bevorzugt eine Binde, eine Einlage, eine Windel oder eine Periodenunterwäsche ist.
